# EUROPÄISCHE PATENTSCHRIFT

(11) **EP 2 166 336 B1**
(45) Veröffentlichungstag und Bekanntmachung des Hinweises auf die Patenterteilung: **16.11.2011**
(21) Anmeldenummer: 08016455.1
(22) Anmeldetag: 18.09.2008
(51) Int. Cl.: G01N 9/00, G01N 9/18, G01N 33/28, G01F 23/72

(54) **Verfahren zum Überwachen der Qualität eines alkoholhaltigen Kraftstoffs in einem Lagertank**
Method for monitoring the quality of a fuel containing alcohol in a storage tank
Procédé de surveillance de la qualité d'un carburant contenant de l'alcool dans un réservoir de stockage

(43) Veröffentlichungstag der Anmeldung: 24.03.2010
(73) Patentinhaber: FAFNIR GmbH, D-22765 Hamburg (DE)
(72) Erfinder: Maurer, Christian Dr., 22547 Hamburg (DE); Schrittenlacher, Wolfgang Dr., 21075 Hamburg (DE)
(74) Vertreter: UEXKÜLL & STOLBERG

(56) Entgegenhaltungen:
- WO-A-2008/064010
- DE-A1-102006 033 237
- US-A1- 2003 057 969
- US-A1- 2006 169 039

## Beschreibung

Kraftstoffe für Fahrzeuge werden in der Regel an Tankstellen in unterirdischen Tanks gelagert. Zur Kontrolle des Bestandes an Kraftstoffen werden seit Jahren und in zunehmendem Maße elektronische Füllstandmesssysteme verwendet, die den Füllstand an ein Warenwirtschaftssystem der Tankstelle übermitteln. Die Qualitätskontrolle beschränkte sich bisher hauptsächlich auf die Detektion von (z.B. durch Lecks am Tankdom) eingedrungenem Wasser. Dieses Wasser sammelt sich auf Grund der höheren Dichte auf dem Boden des Lagertanks und kann dort nachgewiesen werden, üblicherweise in Verbindung mit einem vorhandenen Füllstandmesssystem. Bei kapazitiven Sonden wird der Unterschied in der Dielektrizitätskonstanten, bei Ultraschallsensoren die Schallgeschwindigkeit und bei magnetostriktiven Sonden mittels eines Trennschichtschwimmers der Dichteunterschied an der Grenzfläche zwischen Wasser und Kraftstoff ausgenutzt.

Kraftstoffe sind eine komplexe Zusammensetzung von unterschiedlichen Substanzen. Eine Kraftstoffmischung muss bestimmte Bedingungen an den Dampfdruck, die Oktanzahl, die Dichte und andere Parameter erfüllen. Die Dichte ist einer der Parameter, die auch in den Normen (z.B. EN 228) festgelegt sind. Mögliche Manipulationen an dem gelagerten Kraftstoff lassen sich mit Hilfe einer Dichtemessung erkennen. Die Kraftstoffdichte kann z.B. durch eine Dichtemessung in Verbindung mit magnetostriktiven Füllstandmessungen bestimmt werden, wie es im Stand der Technik beschrieben ist (siehe US 7 278 311 B1, US 2006/0266113 A1, US 2006/0169039 A1, US 5 253 522, DE 10 2006 033 237 A1).

Seit einigen Jahren sind neue Ottokraftstoffe im Verkehr, deren Eigenschaften durch Zusätze von Ethanol oder Methanol erheblich modifiziert werden. Diese Kraftstoffe müssen nach wie vor die in der EN 228 festgelegten Eigenschaften einhalten, so insbesondere auch einen vorgegebenen Dichtebereich. Weil diese Kraftststoffe jedoch zum Teil erheblich mehr Wasser in Lösung aufnehmen können als herkömmliche Ottokraftstoffe aus Kohlenwasserstoffen, kann sich die Dichte bei höheren Wasserkonzentrationen signifikant verändern. Wenn die Löslichkeitsgrenze überschritten wird, fällt nicht allein das weiter zugemischte Wasser aus, sondern es wird auch der im Kraftstoff gelöste Alkohol mit ausgewaschen. Damit beträgt die Dichte der Ausfallphase, die sich im unteren Bereich (Sumpfbereich) eines Lagertanks ansammelt, nicht mehr ca. 1,0 g/cm³, wie bei Wasser, sondern kann weit darunterliegen. Dies kann dazu führen, dass die bisher eingesetzten Trennschichtschwimmer zur Wassererkennung nicht mehr aufschwimmen und somit der Betreiber der Tankstelle nicht mehr gewarnt wird, dass eine Ausfallphase vorliegt, die einen Hinweis auf eine hohe Wasserkonzentration im Kraftstoff liefert. Mit solchem Kraftstoff betankte Fahrzeuge können stehenbleiben oder sogar geschädigt werden.

In Deutschland ist in dem Biokraftstoffquotengesetz eine Beimischung von Ethanol zu Ottokraftstoff von ca. 5% vorgeschrieben (Bezeichnung E05). Eine weitere Erhöhung dieses Anteiles ist in der Diskussion. Es werden jedoch auch Mischungen wie E20, E50 und E85 auf dem Markt angeboten. In einigen anderen Ländern wird auch Methanol zur Beimischung verwendet. Das Verhalten dieser Kraftstoffe bei Wasserkontamination ist sehr unterschiedlich und führt zu nicht erkennbaren Verunreinigungszuständen mit der Gefahr der Schädigung oder Zerstörung der mit diesen Flüssigkeiten betankten Kraftfahrzeuge.

Die Dokumente WO 2008/064010 und US 2003/057969 befassen sich auch mit der Bestimmung der Qualität von alkoholhaltigen Kraftstoffen, verwenden aber dafür Messungen von elektrischen Größen wie widerstand und/oder Kapazität.

Es ist daher Aufgabe der Erfindung, ein Verfahren zu schaffen, dass die Erkennung von gefährlichen Verunreinigungen mit Wasser auch in alkoholhaltigen Kraftstoffen ermöglicht.

Diese Aufgabe wird gelöst durch ein Verfahren zum Überwachen der Qualität eines alkoholhaltigen Kraftstoffs in einem Lagertank mit den Merkmalen des Anspruchs 1. Vorteilhafte Ausgestaltungen der Erfindung ergeben sich aus den Unteransprüchen.

Bei dem erfindungsgemäßen Verfahren zum Überwachen der Qualität eines alkoholhaltigen Kraftstoffs in einem Lagertank wird die Dichte der im Sumpfbereich des Lagertanks befindlichen Flüssigkeit gemessen und mit vorgegebenen Werten verglichen, die die Dichte einer Wasser und Alkohol enthaltenden Mischphase als Funktion der Zusammensetzung charakterisieren.

Der Sumpfbereich ist der untere Bereich des Lagertanks. In der Regel befindet sich die Ansaugöffnung der Saugeinrichtung, über die der Kraftstoff aus dem Lagertank herausgepumpt wird, oberhalb des Sumpfbereichs.

Der Vergleich des im Sumpfbereich gemessenen Dichtewerts mit den vorgegebenen Werten ermöglicht Rückschlüsse auf die Eigenschaften des Kraftstoffs oberhalb des Sumpfbereichs bzw. bedeutet eine Interpretation oder Bewertung dieser Eigenschaften. Zum Beispiel kann man aus dem Vergleich zwischen der gemessenden Dichte der Flüssigkeit im Sumpfbereich mit den vorgegebenen Werten die Zusammensetzung des alkoholhaltigen Kraftstoffs oberhalb des Sumpfbereichs abschätzen. Hierzu wird das Verhalten des Ausgangskraftstoffes, d.h. des zunächst nicht mit Wasser kontaminierten alkoholhaltigen Kraftstoffs (der allenfalls eine von dem Alkohol nicht trennbare Wasserbeimengung enthält), bei Wasserkontamination berücksichtigt, was zu einer definierten Dichte in einer sich im Sumpfbereich ansammelnden Ausfallphase aus Wasser und Alkohol führen kann. Diese Eigenschaften sind nur teilweise bekannt und müssen gegebenenfalls durch Untersuchungen bestimmt werden, um eine richtige Interpretation der Dichtewerte im Sumpfbereich und richtige Rückschlüsse auf den Kraftstoff oberhalb des Sumpfbereichs zu ermöglichen.

Im vorigen Absatz wurde angenommen, dass die für den Vergleich herangezogene Mischphase eine Ausfallphase ist. Dies ist bei alkoholhaltigen Kraftstoffen der Fall, die keine großen Wassermengen aufnehmen können, so dass beim Überschreiten einer bestimmten Wasserkonzentration eine Phasentrennung stattfindet und sich das überschüssige Wasser im Sumpfbereich des Lagertanks ansammelt und dabei auch einen Alkoholanteil aufnimmt.

Es ist aber auch denkbar, dass es nicht zu einer Phasentrennung kommt, so dass sich im Sumpfbereich nicht eine Ausfallphase ansammelt, sondern die dort befindliche Mischphase mit Wasser und Alkohol auch einen großen Anteil an Kohlenwasserstoffen enthält und repräsentativ für den gesamten Inhalt des Lagertanks ist. In diesem Fall kann man ebenfalls aus einer Dichtemessung, hier im Sumpfbereich, durch einen Vergleich mit vorgegebenen Werten Rückschlüsse auf die Kraftstoffzusammensetzung ziehen, insbesondere die Wasserkonzentration, und die Qualität des Kraftstoffs im Lagertank bewerten.

In dieser Beziehung verhalten sich ethanolhaltige Ottokraftstoffe und methanolhaltige Ottokraftstoffe je nach Alkohol- und Wassergehalt recht unterschiedlich, wie in den weiter unten angeführten Beispielen näher erläutert.

Um die Genauigkeit des erfindungsgemäßen Verfahrens zu erhöhen, wird bei bevorzugten Ausführungsformen die Temperaturabhängigkeit der Dichte berücksichtigt. Dazu kann man an mindestens einer Stelle innerhalb des Lagertanks eine für den Ort einer Dichtemessung charakteristische Temperatur messen, wobei der Vergleich von Messwerten für die Dichte mit vorgegebenen Werten unter Berücksichtigung der gemessenen Temperatur erfolgt.

Dazu kann man zum Beispiel jeweilige für einen Vergleich herangezogene vorgegebene Werte, die die Dichte einer Wasser und Alkohol enthaltenden Mischphase als Funktion der Zusammensetzung charakterisieren, mit der Temperatur parametrisieren und für den Vergleich die vorgegebenen Werte verwenden, die der gemessenen Temperatur entsprechen.

Bei vorteilhaften Ausführungsformen der Erfindung wird die Dichte der im Sumpfbereich des Lagertanks befindlichen Flüssigkeit mit einer magnetostriktiven Dichtemesseinrichtung gemessen. Eine derartige Dichtemesseinrichtung ist zum Beispiel aus DE 10 2006 033 237 A1 bekannt. Sie weist einen Auftriebskörper, eine an dem Auftriebskörper angreifende Feder, deren elastische Deformation ein Maß für die Auftriebskraft des Auftriebskörpers ist, und einen an dem Auftriebskörper angeordneten Magneten auf. Die elastische Deformation der Feder wird mittels eines magnetostriktiven Positionsmesssystems erfasst. Bei dieser Einrichtung befindet sich der Auftriebskörper ganz in der zu messenden Flüssigkeit, wobei die von der Dichte der Flüssigkeit abhängende Auftriebskraft quasi mit einer Federwaage gemessen wird. Vorzugsweise wird die Differenz der Positionen des Magneten und eines fixierten Referenzmagneten entlang eines Messdrahts des magnetostriktiven Positionsmesssystems als Maß für die zu bestimmende elastische Deformation der Feder verwendet.

Wie eingangs erwähnt, ist in einem Lagertank für Kraftstoff häufig ein magnetostriktives Füllstandmesssystem installiert, bei dem die Position eines an der Oberfläche des Kraftstoffs schwimmenden, mit einem Magneten versehenen Schwimmers mit Hilfe eines magnetostriktiven Positionsmesssystems erfasst wird. In einem solchen Fall lässt sich das somit vorhandene magnetostriktive Positionsmesssystems auch für eine Dichtemessung im Sumpfbereich des Lagertanks nutzen. Denn mit einem einzigen magnetostriktiven Messdraht und einer zugehörigen Auswerteelektronik kann man die Positionen von zwei und sogar mehr als zwei Magneten entlang des Messdrahts bestimmen.

Dies eröffnet auch die Möglichkeit, die Dichte des im Lagertank befindlichen alkoholhaltigen Kraftstoffs oberhalb des Sumpfbereichs mit einer weiteren magnetostriktiven Dichtemesseinrichtung zu messen, die wiederum einen Auftriebskörper, eine an dem Auftriebskörper angreifende Feder, deren elastische Deformation ein Maß für die Auftriebskraft des Auftriebskörpers ist, und einen an dem Auftriebskörper angeordneten Magneten aufweist. Die elastische Deformation der Feder wird mittels desselben magnetostriktiven Positionsmesssystems erfasst, das auch für die Messung der Dichte der im Sumpfbereich befindlichen Flüssigkeit verwendet wird. Die Messwerte für die Dichte des im Lagertank befindlichen alkoholhaltigen Kraftstoffs oberhalb des Sumpfbereichs können ebenfalls mit vorgegebenen Werten verglichen werden, die die Dichte einer Mischphase des alkoholhaltigen Kraftststoffs mit Wasser als Funktion der Wasserkonzentration charakterisieren. Auf diese Weise lassen sich also Messwerte im Sumpfbereich und oberhalb davon gewinnen, was eine besonders umfassende Charakterisierung der Eigenschaften des Kraftstoffs zum Messzeitpunkt ermöglicht.

Das erfindungsgemäße Verfahren lässt sich zur kontinuierlichen Überwachung der Kraftstoffqualität nutzen, wenn die Dichte der im Sumpfbereich des Lagertanks befindlichen Flüssigkeit fortlaufend gemessen und mit den vorgegebenen Werten (also z.B. der Dichte einer Wasser/Ethanol-Mischung oder der Dichte einer Wasser/Methanol-Mischung als Funktion der Wasserkonzentration) verglichen wird. Vorzugsweise erfolgt durch Speicherung der Messwerte eine Protokollierung. Wenn auch die Dichte des Kraftstoffs oberhalb des Sumpfbereichs gemessen wird, können diese Werte für die Überwachung in ähnlicher Weise herangezogen werden. Ein das Vergleichsergebnis charakterisierendes Signal (das also z.B. angibt, dass die gemessene Dichte einer bestimmten Wasserkonzentration im Sumpfbereich entspricht) kann z.B. über eine digitale Schnittstelle an ein Reportsystem ausgegeben werden. Wenn das Vergleichsergebnis auf das Vorliegen einer Ausfallphase hinweist, wird vorzugsweise ein Warnsignal erzeugt.

Wie bereits erwähnt, ist nicht bei allen alkoholhaltigen Kraftstoffen bei Wasserbeimengung eine Ausfallphase zu erwarten. Aber auch in diesem Fall lässt das Ergebnis des Vergleichs einer Dichtemessung im Sumpfbereich mit vorgegebenen Werten Rückschlüsse auf die momentane Kraftstoffqualität zu, wobei vorzugsweise ein das Vergleichsergebnis charakterisierendes Signal über eine digitale Schnittstelle an ein Reportsystem ausgegeben wird und vorzugsweise ein Warnsignal erzeugt wird, wenn das Vergleichsergebnis auf das Vorliegen einer einen vorgegebenen Grenzwert überschreitenden Wasserbeimischung zu dem Kraftstoff hinweist.

Bei dem erfindungsgemäßen Verfahren liefert die Auswertung der Dichtedaten im Sumpfbereich die Möglichkeit, nicht nur Abweichungen in der Produktqualität des gelagerten Kraftstoffes zu bestimmen, sondern auch komplexe Ausfallphasen zu diagnostizieren, die bei neuartigen alkoholhaltigen Kraftstoffen auftreten können. Die bisher üblichen Trennschichtschwimmer können dafür nicht genutzt werden, da sie keine quantitativen Dichtewerte liefern, sondern nur dann aufschwimmen, wenn eine vorgegebene Dichtedifferenz überschritten wird; da in Ausfallphasen die Dichte häufig signifikant kleiner als die Dichte von Wasser ist, würde bei derartigen Trennschichtschwimmern oftmals sogar die Differenz der Dichten im Kraftstoff und in der Ausfallphase gar nicht ausreichen, um ein Aufschwimmen zu bewirken.

Im folgenden wird die Erfindung anhand von Ausführungsbeispielen weiter erläutert. Die Zeichnungen zeigen in
- Figur 1: einen Längsschnitt durch einen Teil eines Lagertanks für Kraftstoff mit einem magnetostriktiven Positions- messsytem für die Füllstandmessung, das im unteren Be- reich eine bei der Durchführung des erfindungsgemäßen Verfahrens verwendete Dichtemesseinrichtung trägt, und
- Figur 2 eine: Ausschnittsvergrößerung aus Figur 1 (bei etwas verändertem Füllstand im Sumpfbereich des Lagertanks), die die Dichtemesseinrichtung genauer zeigt.

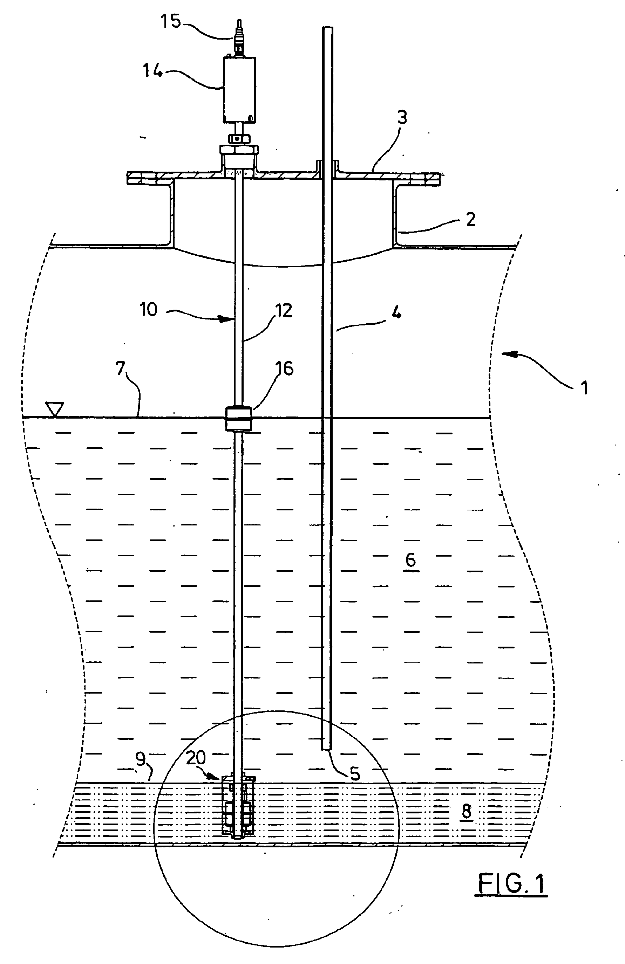

In Figur 1 ist ein Teilbereich eines Lagertanks 1 für Kraftstoff im Längsschnitt dargestellt. Der Lagertank 1 besitzt einen Dom 2 mit einem Deckel 3, durch den ein Saugrohr 4 mit einem unteren Ende 5 hindurchgeführt ist. Das Saugrohr 4 dient zum Abpumpen des Kraftstoffs 6, dessen Füllstandpegel bei 7 liegt. Das untere Ende 5 des Saugrohrs 4 befindet sich oberhalb eines Sumpfbereichs 8 (Pegel 9), um im normalen Betrieb des Lagertanks 1 keine Flüssigkeit aus dem Sumpfbereich 8 anzusaugen.

Der Lagertank 1 ist mit einem herkömmlichen magnetostriktiven Positionsmesssysem 10 zur Füllstandmessung ausgerüstet.

Das Positionsmesssystem 10 weist ein Schutzrohr 12 auf, das mit seinem unteren Ende fast bis zum Boden des Lagertanks 1 reicht. Im Inneren des Schutzrohrs 12 verläuft auf dessen Längsachse ein Messdraht des magnetostriktiven Positionsmesssystems, der zu einer Elektronikeinheit 14 geführt ist. Die Elektronikeinheit 14 enthält an ihrer Oberseite einen Anschluss 15, um ein Signalkabel anzuschließen, das mit einer externen Auswerte- und Steuereinrichtung verbunden werden kann.

Magnetostriktive Messsystems sind bekannt, wie eingangs erläutert. Dabei wird ein Permanentmagnet als Positionsgeber genutzt. Durch den Magnetostriktionseffekt werden in einem magnetostriktiven Wellenleiter, hier dem in dem Schutzrohr 12 enthaltenen Messdraht, Ultraschallwellen erzeugt. Die Laufzeit dieser Ultraschallwellen kann mit hoher Präzision gemessen werden, wozu hier die Elektronikeinheit 14 genutzt wird, so dass sich die Position des Permanentmagneten z.B. bis auf 10 µm genau reproduzierbar bestimmen lässt. Es ist auch möglich, eine größere Anzahl von solchen magnetischen Positionsgebern auf einem magnetostriktiven Wellenleiter gleichzeitig oder praktisch gleichzeitig zu messen, was bereits bei einem Füllstand- und Trennschichtmesssystem in herkömmlicher Ausgestaltung ausgenutzt wird.

Der Füllstandpegel 7 des Kraftstoffs 6 wird mit einem Füllstandschwimmer 16 gemessen, der auf dem Schutzrohr 12 geführt ist und an der Oberfläche des Kraftsoffs 6 schwimmt. Im Inneren des Füllstandschwimmers 16 befindet sich ein Permanentmagnet, mit dessen Hilfe über den Magnetostriktionseffekt die Position des Füllstandschwimmers 16, also der Füllstand in dem Lagertank 1, bestimmt werden kann.

Um die Dichte der Flüssigkeit im Sumpfbereich 8. des Lagertank. 1 messen zu können, wird ebenfalls das magnetostriktive Positionsmessystem 10 genutzt. Dazu ist im Sumpfbereich 8 eine Dichtemesseinrichtung 20 an dem Schutzrohr 12 angebracht.

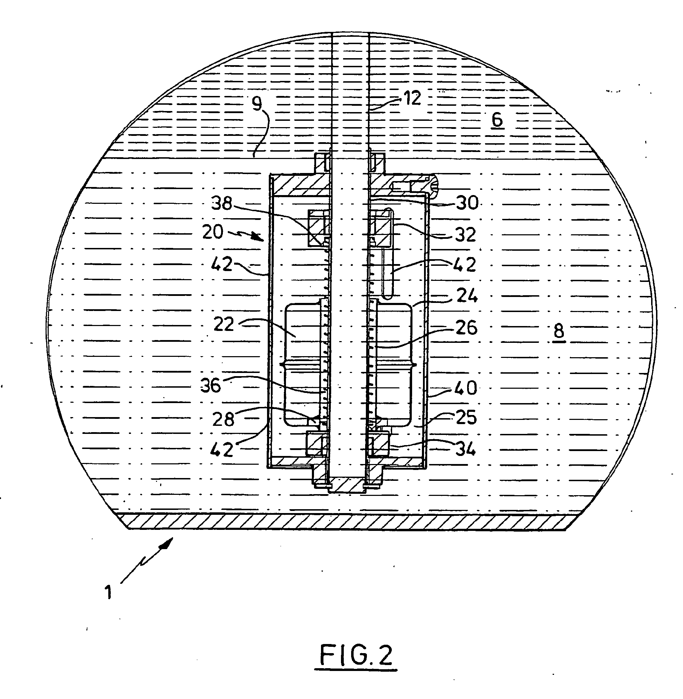

Die Dichtemesseinrichtung 20, deren Prinzip auch in der DE 10 2006 033 237 A1 ausführlich beschrieben ist, ist in Figur 2 in einem vergrößerten Längsschnitt dargestellt.

Ein Auftriebskörper 22 mit einem oberen Ende 24 und einem unteren Ende 25, der im Ausführungsbeispiel rotationssymmetrisch um seine Längsachse gebaut ist, weist entlang der Längsachse eine zylindrische Aussparung 26 auf. In der Nähe des unteren Endes 25 enthält der Innenraum des Auftriebskörpers 22 einen Magneten 28, der ein Permanentmagnet ist und als Positionsgeber in dem magnetostriktiven Positionsmesssystem 10 dient.

Der Auftriebskörper 22 ist längsverschiebbar auf einem Führungsrohr 30 gelagert und kann sich zwischen einem oberen Anschlag 32 und einem unteren Anschlag 34 bewegen. Der Innendurchmesser des Führungsrohrs 30 ist geringfügig größer als der Außendurchmesser des Schutzrohrs 12.

Im Bereich seines unteren Endes 25 hat der Auftriebskörper 22 eine Öffnung, deren Durchmesser geringfügig größer ist als der Außendurchmesser des Führungsrohrs 30, aber kleiner als der Innendurchmesser der im Ausführungsbeispiel zylindrischen Aussparung 26. Dadurch ist ein Vorsprung ausgebildet, an dem sich eine Schraubenfeder 36 mit ihrem unteren Ende abstützen kann. Das obere Ende der Schraubenfeder 36 liegt an dem oberen Anschlag 32 an.

Der obere Anschlag 32 enthält einen Referenzmagneten 38.

Im Ausführungsbeispiel ist die Auftriebskraft des Auftriebskörpers 22, wenn er in die Flüssigkeit im Sumpfbereich 8 des. Lagertanks 1 vollständig eingetaucht ist, größer als sein Gewicht. Daher bewegt sich der Auftriebskörper 22 von dem unteren Anschlag 34 weg nach oben, wird aber durch die dabei zusammengedrückte Schraubenfeder 36 an einer weiteren Aufwärtsbewegung gehindert. Es stellt sich also ein Gleichgewichtszustand zwischen der Auftriebskraft einerseits und der Gewichtskraft sowie der Federkraft andererseits ein. Je größer die Dichte des Flüssigkeit (genauer gesagt, je größer ihr spezifisches Gewicht), um so größer ist die Auftriebskraft, d.h. um so stärker wird die Schraubenfeder 36 zusammengedrückt. Die Gleichgewichtsposition des Auftriebskörpers 22 kann mit Hilfe des magnetostriktiven Positionsmesssystems 10 über den als Positionsgeber dienenden Magneten 28 ermittelt werden.

Die Dichtemesseinrichtung 20 ist von einem Schutzgehäuse 40 umgeben, das an dem Führungsrohr 30 befestigt ist und vor Flüssigkeitsbewegungen und Verschmutzungen schützt. Damit die Flüssigkeit ins Innere des Schutzgehäuses 40 eintreten kann, sind Öffnungen 42 in der Gehäusewandung vorgesehen, von denen in Figur 2 zwei im Längsschnitt und eine (außerhalb der Papierebene) hinter dem oberen Anschlag 32 befindlich dargestellt sind. Das Führungsrohr 30 ist an dem Schutzrohr 12 befestigt.

Der Referenzmagnet 38 kann ebenfalls als Positionsgeber in dem magnetostriktiven Positionsmesssystem 10 benutzt werden. Daher kann die Dichtemesseinrichtung 20 verwendet werden, ohne dass Kalibrationsmessungen für die absolute Höhe des Magneten 28 durchgeführt werden müssen. Die elastische Deformation der Feder 36, die aufgrund des erläuterten Gleichgewichts ein Maß für die Dichte der Flüssigkeit im Sumpfbereich 8 ist, ergibt sich aus der Differenz der Positionen des Magneten 28 und des Referenzmagneten 38.

Da das magnetostriktive Positionsmesssystem 10 in der Lage ist, die Positionen mehrerer Magnete zu erfassen, kann in dem Lagertank 1 eine weitere Dichtemesseinrichtung oberhalb des Sumpfbereichs 8 vorgesehen sein, um auch die Dichte des Kraftstoffs 6 zu erfassen. Diese Dichtemesseinrichtung ist vorzugsweise von der gleichen Bauart wie die Dichtemesseinrichtung 20, wobei eine Feinabstimmung an den zu messenden Dichtebereich über den Auftriebskörper und/oder die Härte der Schraubenfeder erfolgen kann. Für alle Positionsmessungen lässt sich die Elektronikeinheit 14 nutzen.

Ferner sind in dem Lagertank 1 Temperaturfühler im Sumpfbereich 8 sowie oberhalb des Pegels 9 vorgesehen, die vorzugsweise an den Dichtemesseinrichtungen angeordnet sind. Die Signal- und Versorgungsleitungen der Temperaturfühler können ebenfalls zu der Elektronikeinheit 14 geführt werden.

Das erfindungsgemäße Verfahren kann mit Hilfe der von den Dichtemesseinrichtungen gewonnenen Messwerte durchgeführt werden, wie eingangs erläutert. Dazu lassen sich auch externe Rechner verwenden, die über eine Schnittstelle mit dem Anschluss 15 der Elektronikeinheit 14 verbunden sind und auf denen die gemessenen Dichtewerte mit den vorgegebenen Werten verglichen und bewertet werden.

Es folgen zwei Beispiele, die den Ablauf der Verfahren weiter veranschaulichen.

### Beispiel 1

Bei einem alkoholhaltigen Ottokraftstoff mit einer Ethanolbeimischung zu Kohlenwasserstoffen steigt die Löslichkeitsgrenze für Wasser mit zunehmendem Ethanolgehalt an. Wird diese Grenze überschritten, fällt eine Flüssigkeit am Boden des Lagertanks (Sumpfbereich) aus, die Wasser und einen Teil des zuvor in dem Kraftstoff gelösten Ethanols enthält. Die Dichte dieses Gemisches (Ausfallphase) ist nicht als lineare Interpolation über das Verhältnis der Ausgangsvolumina und die Dichten von Wasser (ca. 1,0 g/cm³) und Ethanol (ca. 0,79 g/cm³) zu bestimmen, sondern es muß ein Schrumpfungsfaktor berücksichtigt werden, der bis zu 4% ausmachen kann. Die Dichte in der Ausfallphase nimmt mit zunehmendem Wasseranteil zu, so dass der Unterschied zu dem Ausgangsottokraftstoff (0,72 bis 0,78 g/cm³) immer größer wird.

Mit dem erfindungsgemäßen Verfahren könnte selbst der kleine Dichteunterschied zwischen reinem Ethanol (was in der Praxis nicht vorkommt, da immer ein Wasseranteil vorhanden ist) und der oberen Dichtegrenze des Ottokraftstoffs noch detektiert werden. Dabei muss die bekannte Temperaturabhängigkeit der Dichten der Flüssigkeiten berücksichtigt werden. Der Temperaturwert in der Flüssigkeit kann mittels eines Temperaturfühlers an dem magnetostriktiven Füllstandmesssystem geliefert werden, das für die Dichtemesseinrichtung genutzt wird.

In dem für das Verfahren verwendeten Auswertesystem wird die in dem Lagerbehälter gelagerte Kraftstoffqualität hinterlegt. Damit ist der zulässige zu erwartendende Dichtebereich und dessen Temperaturabhängigkeit bekannt. Bei ethanolhaltigen Ottokraftstoffen mit geringem Ethanolanteil (bis ca. 25%) kann damit eine durch Wasserkontamination hervorgerufene Ausfallphase sicher erkannt werden.

Bei höheren Ethanolkonzentrationen (>25%) wächst die Lösungsfähigkeit für Wasser stark an, d.h. es bildet sich keine separate Phase (Ausfallphase). Es steigt jedoch mit steigendem Wasseranteil die Dichte der gesamten Mischung an und somit also auch im Sumpfbereich, so dass eine Wasserverunreinigung auch durch eine Dichtemessung im Sumpfbereich erkannt werden kann.

### Beispiel 2

Bei methanolhaltigen Ottokraftstoffen ist das Verhalten bezüglich Wasserkontamination teilweise sehr unterschiedlich. Bei kleineren Methanolkonzentrationen (bis ca. 20%) fällt ebenfalls ein Methanol/Wasser-Gemisch aus, jedoch ist die Menge schon bei kleinen Wasserverunreinigungen deutlich größer. Der Auswaschungseffekt ist also signifikant stärker. Die auftretenden Ausfallphasen sind mit dem erfindungsgemäßen Verfahren zu detektieren.

Bei höheren Methanolkonzentrationen entsteht im Gegensatz zu den Ethanolmischungen bereits bei kleinen Wasserkonzentrationen eine Ausfallphase, deren Dichte in der Nähe der Dichte des Methanols liegt. Diese Phase kann auch noch einen Anteil an Kohlenwasserstoffen aus dem Kraftstoff enthalten. Auch diese Phase lässt sich durch die Dichtemessung im Sumpfbereich erkennen. Höhere Wasserkonzentrationen führen hier ebenfalls zu einer Zunahme der Ausfallphase und zu einem Anstieg ihrer Dichte.

## Patentansprüche

1. Verfahren zum Überwachen der Qualität eines alkoholhaltigen Kraftstoffs in einem Lagertank (1), der einen Sumpfbereich (8) aufweist, **dadurch gekennzeichnet, dass** die Dichte der im Sumpfbereich (8) des Lagertanks (1) befindlichen Flüssigkeit gemessen und mit vorgegebenen Werten verglichen wird, die die Dichte einer Wasser und Alkohol enthaltenden Mischphase als Funktion der Zusammensetzung charakterisieren.

2. Verfahren nach Anspruch 1, **dadurch gekennzeichnet, dass** die Mischphase eine Ausfallphase ist.

3. Verfahren nach Anspruch 1 oder 2, **dadurch gekennzeichnet, dass** aus dem Vergleich zwischen der gemessenen Dichte der Flüssigkeit im Sumpfbereich (8) mit den vorgegebenen Werten die Zusammensetzung des alkoholhaltigen Kraftstoffs oberhalb des Sumpfbereichs (8) abgeschätzt wird.

4. Verfahren nach einem der Ansprüche 1 bis 3, **dadurch gekennzeichnet, dass** die Dichte der im Sumpfbereich (8) des Lagertanks (1) befindlichen Flüssigkeit mit einer magnetostriktiven Dichtemesseinrichtung (20) gemessen wird, die einen Auftriebskörper (22), eine an dem Auftriebskörper (22) angreifende Feder (36), deren elastische Deformation ein Maß für die Auftriebskraft des Auftriebskörpers (22) ist, und einen an dem Auftriebskörper (22) angeordneten Magneten (28) aufweist, wobei die elastische Deformation der Feder (36) mittels eines magnetostriktiven Positionsmesssystems (10) erfasst wird.

5. Verfahren nach Anspruch 4, **dadurch gekennzeichnet, dass** die Differenz der Positionen des Magneten (28) und eines fixierten Referenzmagneten (38) entlang eines Messdrahts des magnetostriktiven Positionsmesssystems (10) als Maß für die zu bestimmende elastische Deformation der Feder (36) verwendet wird.

6. Verfahren nach Anspruch 4 oder 5, **dadurch gekennzeichnet, dass** mit Hilfe des magnetostriktiven Positionsmesssystems (10) auch der Füllstand des alkoholhaltigen Kraftstoffs in dem Lagertank (1) gemessen wird.

7. Verfahren nach einem der Ansprüche 4 bis 6, **dadurch gekennzeichnet, dass** die Dichte des im Lagertank (1) befindlichen alkoholhaltigen Kraftstoffs (6) oberhalb des Sumpfbereichs (8) mit einer weiteren magnetostriktiven Dichtemesseinrichtung gemessen wird, die einen Auftriebskörper, eine an dem Auftriebskörper angreifende Feder, deren elastische Deformation ein Maß für die Auftriebskraft des Auftriebskörpers ist, und einen an dem Auftriebskörper angeordneten Magneten aufweist, wobei die elastische Deformation der Feder mittels desselben magnetostriktiven Positionsmesssystems (10) erfasst wird, das auch für die Messung der Dichte der im Sumpfbereich (8) befindlichen Flüssigkeit verwendet wird.

8. Verfahren nach Anspruch 7, **dadurch gekennzeichnet, dass** die Messwerte für die Dichte des im Lagertank (1) befindlichen alkoholhaltigen Kraftstoffs (6) oberhalb des Sumpfbereichs (8) mit vorgegebenen Werten verglichen werden, die die Dichte einer Mischphase des alkoholhaltigen Kraftstoffs mit Wasser als Funktion der Wasserkonzentration charakterisieren.

9. Verfahren nach einem der Ansprüche 1 bis 8, **dadurch gekennzeichnet, dass** an mindestens einer Stelle innerhalb des Lagertanks (1) eine für den Ort einer Dichtemessung charakteristische Temperatur gemessen wird und der Vergleich von Messwerten für die Dichte mit vorgegebenen Werten unter Berücksichtigung der gemessenen Temperatur erfolgt.

10. Verfahren nach Anspruch 9, **dadurch gekennzeichnet, dass** jeweilige für einen Vergleich herangezogene vorgegebene Werte mit der Temperatur parametrisiert sind und dass für den Vergleich die vorgegebenen Werte verwendet werden, die der gemessenen Temperatur entsprechen.

11. Verfahren nach einem der Ansprüche 1 bis 10, **dadurch gekennzeichnet, dass** der Kraftstoff ein ethanolhaltiger Ottokraftstoff ist.

12. Verfahren nach einem der Ansprüche 1 bis 11, **dadurch gekennzeichnet, dass** der Kraftstoff ein methanolhaltiger Ottokraftstoff ist.

13. Verfahren nach einem der Ansprüche 1 bis 12, **dadurch gekennzeichnet, dass** zumindest die Dichte der im Sumpfbereich (8) des Lagertanks (1) befindlichen Flüssigkeit fortlaufend gemessen und mit den vorgegebenen Werten verglichen wird.

14. Verfahren nach einem der Ansprüche 1 bis 13 in Verbindung mit Anspruch 2, **dadurch gekennzeichnet, dass** ein das Vergleichsergebnis charakterisierendes Signal über eine digitale Schnittstelle an ein Reportsystem ausgegeben wird und dass vorzugsweise ein Warnsignal erzeugt wird, wenn das Vergleichsergebnis auf das Vorliegen einer Ausfallphase hinweist.

15. Verfahren nach einem der Ansprüche 1 oder 3 bis 13 bei einem Kraftstoff, der keine Ausfallphase erwarten lässt, **dadurch gekennzeichnet, dass** ein das Vergleichsergebnis charakterisierendes Signal über eine digitale Schnittstelle an ein Reportsystem ausgegeben wird und dass vorzugsweise ein Warnsignal erzeugt wird, wenn das Vergleichsergebnis auf das Vorliegen einer einen vorgegebenen Grenzwert überschreitenden Wasserbeimischung zu dem Kraftstoff hinweist.

## Claims

1. Method for monitoring the quality of an alcohol-containing fuel in a storage tank (1) which comprises a sump area (8), **characterised in that** the density of the liquid contained in the sump area (8) of the storage tank (1) is measured and compared with predetermined values which are characteristic of the density of a mixed phase containing water and alcohol as a function of the composition.

2. Method according to claim 1, **characterised in that** the mixed phase is a faulty phase.

3. Method according to claim 1 or 2, **characterised in that** from the comparison of the measured density of the liquid in the sump area (8) with the predetermined values the composition of the alcohol-containing fuel above the sump area (8) is estimated.

4. Method according to one of claims 1 to 3, **characterised in that** the density of the liquid located in the sump area (8) of the storage tank (1) is measured by a magnetostrictive density measuring device (20), which comprises a buoyancy body (22), a spring (36) acting on the buoyancy body (22), the elastic deformation of which is a measure of the buoyancy force of the buoyancy body (22) and a magnet (28) arranged on the buoyancy body (22), wherein the elastic deformation of the spring (36) is determined by means of a magnetostrictive position measuring system (10).

5. Method according to claim 4, **characterised in that** the difference in the positions of the magnet (28) and a fixed reference magnet (38) along a measuring wire of the magnetostrictive position measuring system (10) is used as a measure for the elastic deformation of the spring (36) to be determined.

6. Method according to claim 4 or 5, **characterised in that** by means of the magnetostrictive position measuring system (10) also the filling level of the alcohol-containing fuel in the storage tank (1) is measured.

7. Method according to one of claims 4 to 6, **characterised in that** the density of the alcohol-containing fuel (6) located in the storage tank (1) is measured above the sump area (8) by an additional magnetostrictive density measuring device, which comprises a buoyancy body, a spring acting on the buoyancy body, the elastic deformation of which is a measure of the buoyancy force of the buoyancy body and a magnet arranged on the buoyancy body, wherein the elastic deformation of the spring is determined by means of the same magnetostrictive position measuring system (10), which is also used for measuring the density of the liquid located in the sump area (8).

8. Method according to claim 7, **characterised in that** the measurement values for the density of the alcohol-containing fuel (6) in the storage tank (1) above the sump area (8) are compared with predetermined values, which are characteristic of the density of a mixed phase of the alcohol-containing fuel with water as a function of the water concentration.

9. Method according to one of claims 1 to 8, **characterised in that** in at least one point inside the storage tank (1) a temperature characteristic of the site of a density measurement is measured and the comparison of measurement values for the density with predetermined values is performed taking into account the measured temperature.

10. Method according to claim 9, **characterised in that** the respective values used for comparison are parameterised with the temperature, and **in that** for the comparison predetermined values are used which correspond to the measured temperature.

11. Method according to one of claims 1 to 10, **characterised in that** the fuel is an ethanol-containing petrol.

12. Method according to one of claims 1 to 11, **characterised in that** the fuel is a methanol-containing petrol.

13. Method according to one of claims 1 to 12, **characterised in that** at least the density of the liquid located in the sump area (8) of the storage tank (1) is measured continually and compared with the predetermined values.

14. Method according to one of claims 1 to 13 in connection with claim 2, **characterised in that** a signal characterising the comparison result is sent via a digital interface to a report system, and **in that** preferably a warning signal is generated when the comparison result indicates the presence of a faulty phase.

15. Method according to one of claims 1 or 3 to 13 with a fuel which would not be expected to have a faulty phase, **characterised in that** a signal characterising the comparison result is sent via a digital interface to a report system, and **in that** preferably a warning signal is generated when the comparison result indicates the presence of a water admixture with the fuel which exceeds a predetermined limit value.

## Revendications

1. Procédé de surveillance de la qualité d'un carburant contenant de l'alcool dans un réservoir de stockage (1) qui présente une zone de puisard (8), **caractérisé en ce que** l'on mesure la densité du liquide se trouvant dans la zone de puisard (8) du réservoir de stockage (1) et on la compare avec des valeurs prédéfinies qui caractérisent la densité d'une phase mixte contenant de l'eau et de l'alcool, en tant que fonction de la composition.

2. Procédé selon la revendication 1, **caractérisé en ce que** la phase mixte est une phase décantée.

3. Procédé selon la revendication 1 ou 2, **caractérisé en ce que** l'on évalue, à partir de la comparaison de la densité mesurée du liquide se trouvant dans la zone de puisard (8) avec les valeurs prédéfinies, la composition du carburant contenant de l'alcool, se trouvant au-dessus de la zone de puisard (8).

4. Procédé selon une des revendications 1 à 3, **caractérisé en ce que** la densité du liquide se trouvant dans la zone de puisard (8) du réservoir de stockage (1) est mesurée avec un densimètre (20) magnétostrictif qui présente un flotteur (22), un ressort (36) qui est en prise avec le flotteur (22) et dont la déformation élastique constitue une mesure pour la poussée d'Archimède du flotteur (22), et un aimant (28) disposé sur le flotteur (22), la déformation élastique du ressort (36) étant détectée au moyen d'un système de mesure de position (10) magnétostrictif.

5. Procédé selon la revendication 4, **caractérisé en ce que** la différence des positions de l'aimant (28) et d'un aimant de référence (38) fixe, le long d'un fil potentiométrique du système de mesure de position (10) magnétostrictif, est utilisée comme mesure pour la déformation élastique du ressort (36) qui doit être déterminée.

6. Procédé selon la revendication 4 ou 5, **caractérisé en ce que** le système de mesure de position (10) magnétostrictif permet également de mesurer le niveau du carburant contenant de l'alcool, dans le réservoir de stockage (1).

7. Procédé selon une des revendications 4 à 6, **caractérisé en ce que** la densité du carburant (6) contenant de l'alcool, se trouvant dans le réservoir de stockage (1), est mesurée au-dessus de la zone de puisard (8) avec un deuxième densimètre magnétostrictif qui présente un flotteur, un ressort qui est en prise avec le flotteur et dont la déformation élastique constitue une mesure pour la poussée d'Archimède du flotteur, et un aimant disposé sur le flotteur, la déformation élastique du ressort étant détectée au moyen du même système de mesure de position (10) magnétostrictif que celui qui est utilisé pour mesurer la densité du liquide se trouvant dans la zone de puisard (8).

8. Procédé selon la revendication 7, **caractérisé en ce que** les valeurs mesurées pour la densité du carburant (6) contenant de l'alcool, se trouvant dans le réservoir de stockage (1), au-dessus de la zone de puisard, sont comparées avec des valeurs prédéfinies qui caractérisent la densité d'une phase mixte du carburant, contenant de l'alcool, avec de l'eau, en tant que fonction de la concentration d'eau.

9. Procédé selon une des revendications 1 à 8, **caractérisé en ce que** l'on mesure, à au moins un endroit dans le réservoir de stockage (1), une température caractéristique du lieu d'une densimétrie, et la comparaison de valeurs mesurées pour la densité, avec des valeurs prédéfinies, est effectuée en tenant compte de la température mesurée.

10. Procédé selon la revendication 9, **caractérisé en ce que** des valeurs prédéfinies respectives, utilisées pour une comparaison, sont paramétrées avec la température, et **en ce que** l'on utilise, pour la comparaison, les valeurs prédéfinies qui correspondent à la température mesurée.

11. Procédé selon une des revendications 1 à 10, **caractérisé en ce que** le carburant est un carburant pour moteurs à étincelles, contenant de l'éthanol.

12. Procédé selon une des revendications 1 à 11, **caractérisé en ce que** le carburant est un carburant pour moteurs à étincelles, contenant du méthanol.

13. Procédé selon une des revendications 1 à 12, **caractérisé en ce que** l'on mesure en continu au moins la densité du liquide se trouvant dans la zone de puisard (8) du réservoir de stockage (1) et on la compare avec les valeurs prédéfinies.

14. Procédé selon une des revendications 1 à 13 en relation avec la revendication 2, **caractérisé en ce qu'**un signal caractérisant le résultat de la comparaison est transmis à un système de rapport, via une interface numérique, et **en ce que**, de préférence, un signal avertisseur est produit lorsque le résultat de la comparaison indique la présence d'une phase décantée.

15. Procédé selon une des revendications 1 ou 3 à 13 pour un carburant pour lequel on ne s'attend pas à une phase décantée, **caractérisé en ce qu'**un signal caractérisant le résultat de la comparaison est transmis à un système de rapport, via une interface numérique, et **en ce que**, de préférence, un signal avertisseur est produit lorsque le résultat de la comparaison indique la présence d'eau mélangée au carburant, dépassant une valeur limite prédéfinie.
